# EUROPEAN PATENT SPECIFICATION

(11) **EP 0 595 908 B1**
(45) Date of publication and mention of the grant of the patent: **17.04.1996**
(21) Application number: 92915719.6
(22) Date of filing: 25.06.1992
(51) Int. Cl.: A61M 16/06, A62B 18/02

(54) **ANAESTHETIC MASK FOR INFANTS**
NARKOSEMASKER FÜR KINDER
MASQUE ANESTHESIQUE POUR ENFANTS

(30) Priority: 19.07.1991 SE 9102205
(43) Date of publication of application: 11.05.1994
(73) Proprietor: DAHLSTRAND, Monika, S-371 37 Karlskrona (SE)
(72) Inventor: DAHLSTRAND, Monika, S-371 37 Karlskrona (SE)
(74) Representative: Winblad, Hans Peter
(86) International application number: PCT/SE92/00470
(87) International publication number: WO 93/01854

(56) References cited:
- US-A- 4 520 809
- US-A- 4 896 666

## Description

The invention relates to an anaesthesia mask for infants, comprising a central body section with a through-opening for connection of an anaesthesia tube to the mask, and a bowl-shaped elastic wall projecting from the body section which is shaped so as sealing surround the facial region of the child around the nose and mouth.

In order to anaesthetize infants, particularly babies, an anaesthesia mask of the above-mentioned type is often used which, during administration of the anaesthetic, is continuously pressed against the child's face by a nurse in order to maintain sealed contact. Such a mask is often seen by the child as a threat due to perceived difficulty in breathing in the mask through the nose. This disturbs the child so that anaesthetizing becomes much more difficult.

An anaesthesia administering device is known from EP-A-0 085 639 which includes a teat-shaped suction piece with which the child can suck a nozzle opening for the anaesthesia gas towards himself, the nozzle being formed on the outside of the teat and positioned in front of the nose. The gas can hereby be partially inhaled by the child, though the majority of the gas flows out to the surrounding air. This known device is intended to achieve a first phase of anaesthetising, whereafter a normal anaesthesia mask is connected and placed over the face for subsequent anaesthetizing (c.f. page 4, lines 26-32; page 5, line 1 and 2 of the EP publication).

The claimed mask is an anaesthesia mask for infants which the child will happily accept so as to achieve a sealing gradual contact of the outer peripheral edge of the bowl-shaped wall of the mask against the face of the child around the nose and mouth so as to prevent leakage of anaesthesia gas around the mask and to thereby obtain complete anaesthesia with one single pass. An anesthesia mask according to the preamble of Claim 1 is shown in US-A-4 896 666. The anaesthesia mask according to the invention is characterized in that a teat-shaped suction piece is formed unitarily with a section of the wall beneath the through-opening, which suction piece is intended to be inserted into the child's mouth so as to facilitate a sealing gradual contact of the wall's outer peripheral edge against the face by means of the child sucking on the suction piece.

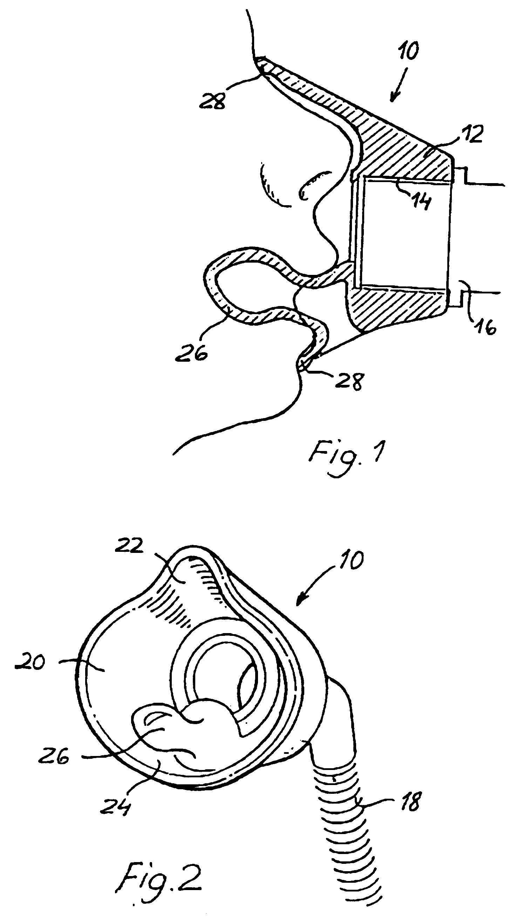

A suitable embodiment of the anaesthesia mask according to the invention will be described in more detail below with reference to the attached drawings in which Fig. 1 shows a cross-section through the mask according to the invention whilst in use, and Fig. 2 is a perspective view from the rear of the mask.

The anaesthesia mask 10 according to the invention which is made from rubber or another suitable elastic material comprises a central body section 12 with a through-opening 14 for a connection nipple 16 which is intended to be connected to an external anaesthesia gas tube 18 in order to direct a flow of gas towards the nostrils. A bowl-shaped wall 20 projects from the central section 12 and presents an upper region 22 which surrounds the child's nose, and a lower section 24 on which an inwardly projecting teat-shaped suction piece 26 is formed. The suction piece is totally sealed and formed in one piece with the lower section 24 of the mask's wall and is intended to be inserted in the child's mouth.

The wall 20 has an outer peripheral edge with a bead 28 on the side of the mask facing the child. The purpose of the bead 28 is to form a sealing contact of the mask against the child's face.

Before the mask is used, a sugar solution or similar may be applied to the teat-shaped suction piece 26, whereafter the suction piece is inserted in the child's mouth so that the child can happily suck on it so that the peripheral edge of the mask is drawn into sealing contact with the face around the nose and mouth (Fig. 1). Anaesthesia gas can thereafter be administered to anaesthetize the child.

## Claims

1. Anaesthesia mask for infants, comprising a central body section (12) with a through-opening (14) for connection of an anaesthesia tube (18) to the mask, and a bowl-shaped elastic wall (20) projecting from the body section (12), which wall is shaped so as to sealing surround the facial region of the child around the nose and mouth, **characterized** in that a teat-shaped suction piece (26) is formed unitarily with a section (24) of the wall (20) beneath the through-opening (14), which suction piece (26) is intended to be inserted in the child's mouth so as to facilitates a sealing gradual contact of the outer peripheral edge (28) of the wall against the face by means of the child sucking on the suction piece.

2. Anaesthesia mask according to claim 1, **characterized** in that the outer peripheral edge of the wall (20) presents an inwardly directed bead (28).

3. Anaesthesia mask according to claim 1 or 2, **characterized** in that the through-opening (14) houses a nipple (16) for connection of the anaesthesia gas tube (18).

4. Anaesthesia mask according to any one of claims 1-3, **characterized** in that the teat-shaped suction piece (26) is completely sealed.

## Patentansprüche

1. Narkosemaske für Kinder, umfassend einen zentralen Körperbereich (12) mit einer durchgehenden Öffnung (14) für ein Verbinden eines Narkoseschlauches (18) mit der Maske und eine schalenförmige elastische Wand (20), welche von dem Körperbereich (12) vorragt, welche Wand so geformt ist, daß sie den Gesichtsbereich des Kindes um die Nase und Mund dichtend umgibt, dadurch gekennzeichnet, daß ein Brustwarzen-artig geformtes Saugstück (26) einstückig mit einem Bereich (24) der Wand (20) unter der durchgehenden Öffnung (14) geformt ist, welches Saugstück (26) in den Mund des Kindes eingeführt werden soll, um einen zunehmenden Dichtkontakt des äußeren Randes (28) der Wand gegen das Gesicht durch das Saugen des Kindes an dem Saugstück zu erleichtern.

2. Narkosemaske nach Anspruch 1, dadurch gekennzeichnet, daß der äußere Rand der Wand (20) einen nach innen gerichteten Wulst (28) aufweist.

3. Narkosemaske nach Anspruch 1 oder 2, dadurch gekennzeichnet, daß die durchgehende Öffnung (14) einen Nippel (16) zum Anschließen des Narkosegasschlauches (18) umgibt.

4. Narkosemaske nach einem der Ansprüche 1 bis 3, dadurch gekennzeichnet, daß das Brustwarzen-artig geformte Saugstück (26) vollständig abgedichtet ist.

## Revendications

1. Masque d'anesthésie pour petits enfants, comprenant un tronçon central (12) de corps ayant une ouverture débouchante (14) destinée au raccordement d'un tube d'anesthésie (18) au masque, et une paroi élastique (20) en forme de cuvette dépassant du tronçon (12) de corps, la paroi ayant une configuration telle qu'elle peut entourer de manière étanche la région du visage de l'enfant autour du nez et de la bouche, caractérisé en ce qu'une pièce d'aspiration (26) en forme de tétine est réalisée en une seule pièce avec un tronçon (24) de la paroi (20) sous l'ouverture débouchante (14), cette pièce d'aspiration (26) étant destinée à être introduite dans la bouche de l'enfant afin qu'un contact étanche progressif du bord périphérique externe (28) de la paroi contre le visage soit facilité lorsque l'enfant suce la pièce d'aspiration.

2. Masque d'anesthésie selon la revendication 1, caractérisé en ce que le bord périphérique externe de la paroi (20) forme un cordon (28) tourné vers l'intérieur.

3. Masque d'anesthésie selon la revendication 1 ou 2, caractérisé en ce que l'ouverture débouchante (14) loge un embout (16) destiné à assurer la connexion du tube (18) de gaz anesthésique.

4. Masque d'anesthésie selon l'une quelconque des revendications 1 à 3, caractérisé en ce que la pièce d'aspiration (26) en forme de tétine est totalement étanche.
